# EUROPEAN PATENT SPECIFICATION

(11) **EP 2 268 292 B1**
(45) Date of publication and mention of the grant of the patent: **27.12.2017**
(21) Application number: 09723788.7
(22) Date of filing: 24.03.2009
(51) Int. Cl.: A61K 33/36, A61P 35/00, A61K 33/24, A61K 31/337

(54) **METHOD AND COMPOSITIONS FOR TREATMENT OF CANCER**
VERFAHREN UND ZUSAMMENSETZUNGEN ZUR KREBSBEHANDLUNG
MÉTHODE ET COMPOSITIONS POUR LE TRAITEMENT DU CANCER

(30) Priority: 23.03.2009 US 408864; 27.03.2008 US 39987 P
(43) Date of publication of application: 05.01.2011
(73) Proprietor: Kominox, Inc., George Town, Grand Cayman KY1-1104 (KY)
(72) Inventor: BURGER, Angelika, Detroit, MI 48201 (US); HENDRIKS, Hans, NL-1443 LR Purmerend (NL); RADEMAKER, Bernardus, NL-9591 AE Onstwedde (NL)
(74) Representative: Müller-Boré & Partner Patentanwälte PartG mbB
(86) International application number: PCT/US2009/038104
(87) International publication number: WO 2009/120697

(56) References cited:
- EP-A1- 1 378 241
- WO-A1-2009/049258
- US-A1- 2004 146 583
- US-A1- 2005 106 262
- LEE T-C ET AL: "SODIUM ARSENITE POTENTIATES THE CLASTOGENICITY AND MUTAGENICITY OF DNA CROSSLINKING AGENTS", ENVIRONMENTAL MUTAGENESIS, JOHN WILEY & SONS, INC, US, vol. 8, no. 1, 1 January 1986 (1986-01-01) , pages 119-128, XP009146966, ISSN: 0192-2521
- SEZGIN C ET AL: "Arsenic trioxide has additive cytotoxic effects on MCF-7 breast cancer cell line with taxanes", TURKISH JOURNAL OF MEDICAL SCIENCES, ANKARA, TR, vol. 32, no. 6, 1 January 2002 (2002-01-01), pages 439-444, XP009146967, ISSN: 1300-0144
- GUO WEI; TANG XIAO-DONG; TANG SHUN; YANG YI: "[Preliminary report of combination chemotherapy including Arsenic trioxide for stage III osteosarcoma and Ewing sarcoma]", ZHONGHUA WAI KE ZA ZHI [CHINESE JOURNAL OF SURGERY], vol. 44, no. 12, 15 June 2006 (2006-06-15) , pages 805-808, XP009146968, China
- WEI WANG ET AL.: 'Experimental study on antitumor effect of arsenic trioxide in combination with cisplatin or doxorubicin on hepatocellular carcinoma.' WORLD J GASTROENTEROL. vol. 7, no. 5, October 2001, pages 702 - 705, XP009144407
- Chuck C.-K. Chao ET AL: "Inhibition by arsenite of anticancer drug cis-diamminedichloroplatinum(II) induced DNA repair and drug resistance in HeLa cells", Environmental Toxicology and Pharmacology, vol. 1, no. 3, 1 May 1996 (1996-05-01), pages 199-205, XP055182425, ISSN: 1382-6689, DOI: 10.1016/1382-6689(96)00010-5
- T.-C. CHOU: "Drug Combination Studies and Their Synergy Quantification Using the Chou-Talalay Method", CANCER RESEARCH, vol. 70, no. 2, 15 January 2010 (2010-01-15), pages 440-446, XP055169871, ISSN: 0008-5472, DOI: 10.1158/0008-5472.CAN-09-1947

## Description

The present invention relates generally to a combination treatment exhibiting synergistic inhibition of the growth and/or proliferation of lung cancer. More particularly, the invention relates to kits for use in the treatment of lung cancer, comprising a first composition comprising sodium meta arsenite and a second composition comprising a cytotoxic anti-cancer agent selected from the group consisting of cisplatin, paclitaxel and docetacel. Further, the invention relates to respective anti-cancer agents for use in the treatment of lung cancer in a human patient, wherein the patient is being, has been or will be subjected to a treatment regime comprising the administration of sodium meta arsenite.

Chemotherapy, the systemic administration of antineoplastic agents that travel throughout the body *via* the blood circulatory system, along with and often in conjunction with surgery and/or radiation treatment, has for years been widely utilized in the treatment of a wide variety of cancers.

Today, there are a variety of antineoplastic agents that have successfully been used in the treatment of cancer. However, the search continues for more efficacious and less toxic agents.

For example, the treatment of cancer patients with platinum coordination complex antineoplastic agents, such as cis-diamminedichloroplatinum (II) (cisplatin) has increased substantially in the last decade. Cisplatin is an antineoplastic agent that has proved useful in the treatment of multiple malignancies including testicular cancer, ovarian cancer, bladder, head and neck and some lung cancers. In particular, cisplatin has been shown to be especially efficacious in the treatment of cancer cells that have long telomeres. Cisplatin is classified as a pro-drug, and once activated, it interacts with nucleophilic sites in DNA, creating predominantly purine-purine intrastrand cross-links. This genotoxic stress activates signal transduction pathways, which in most cells leads to DNA synthesis arrest and programmed cell death. Unfortunately, the initial success of cisplatin chemotherapy is often short-lived, as recurrence of the cancer occurs in many cases. The potential of the platinum-based chemotherapeutics is limited by several factors, including dangerous side effects, limited solubility, and intrinsic or acquired resistance. Thus, new therapies are needed for the treatment of cancer patients.

Like cisplatin, the anti-cancer agents, adriamycin and taxanes, such as paclitaxel, larotaxel, orataxel, tesetaxel and docetaxel, are believed to bind to telomeres or interfere with telomerases. These anti-cancer agents are routinely used to treat bladder, prostate, lung, breast, ovarian and other solid tumors as well as leukemias. However, like cisplatin, their efficacy is often limited by extrinsic factors.

The use of arsenic compounds as therapeutic agents is currently experiencing a revival, particularly in the field of oncology. Studies have shown that certain arsenic compounds are effective against malignancies such as acute promyelocytic leukaemia (APL) (arsenic trioxide) or urogenital cancer (sodium meta arsenite). See Shen, Z.X. et al. Blood 1997;89: 3354-3360; Soignet, S.L. et al. N. Engl. J. Med. 1998; 339: 1341-1348; WO2006121280 A1. Clinical trials of the use of arsenic trioxide in the treatment of APL patients are being conducted in the US and the mechanism(s) of action of various arsenic compounds is being studied to enable rational use of inorganic arsenic. See Chou, W.C. et al. J. Clin. Invest. 2001; 108: 1541-1547; Senior K. Drug Discovery Today 2002; 7: 156-157.

In these clinical trials, it has been reported that arsenic trioxide exerts antitumor effects by activating apoptosis, by induction of reactive oxygen species and by degradation of PML-RARa fusion protein (Chou et al.). Most recently, its efficacy has been linked to the inhibition of the transcription of the reverse transcriptase subunit of the human telomerase gene (hTERT) and subsequently induction of telomere shortening as well as of chromosomal instability (Chou et al. and Senior, *supra*).

Similarly, studies have shown that sodium meta arsenite inactivates transcription of the hTERT gene and is capable of shortening telomeres in human cancer cells, indicating that it is a telomere poison. However, sodium meta arsenite is most effective in the treatment of cancers having short telomeres.

In this context, Chao *et al.* (Chao, C. C.-K.; Environmental Toxicology and Pharmacology, 1; 1996; pp. 199-205) describes the inhibition of cisplatin-induced DNA repair and drug resistance by arsenite in HeLa cells.

Thus, there remains a need for improved compositions for the treatment of cancer and metastasis.

The present invention relates to the following items:
1. A kit for use in the treatment of lung cancer comprising a first composition comprising a therapeutically effective amount of sodium meta arsenite and a second composition comprising a therapeutically effective amount of a cytotoxic anti-cancer agent selected from the group consisting of cisplatin, paclitaxel and docetaxel.
2. The kit according to item 1 wherein the first composition comprises sodium meta arsenite and the second composition comprises cisplatin.
3. The kit according to item 2 wherein the first composition is formulated for oral administration.
4. The kit according to item 1 wherein the first composition comprises sodium meta arsenite and the second composition comprises paclitaxel.
5. An anti-cancer agent selected from the group consisting of cisplatin, paclitaxel and docetaxel for use in the treatment of lung cancer in a human patient, wherein the patient is being, has been or will be subjected to a treatment regime comprising the administration to the patient of sodium meta arsenite.
6. The anti-cancer agent for use according to item 5 wherein the anti-cancer agent is cisplatin.
7. The anti-cancer agent for use according to item 5 wherein the anti-cancer agent is paclitaxel.

Effective treatment of lung cancer and/or prevention of metastasis is achieved by administration of sodium meta arsenite (NaAsO₂ in combination with cisplatin, docetaxel or paclitaxel.

The figures show:

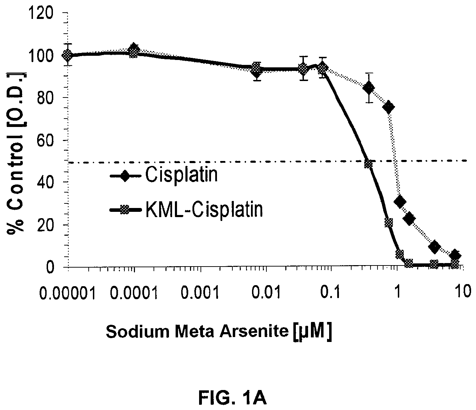

Figure 1A is a graph of the growth curve for single agent cisplatin (diamonds) and the simultaneous combination of cisplatin with sodium meta arsenite (squares) in A549 cells; results are from MTT proliferation assays.

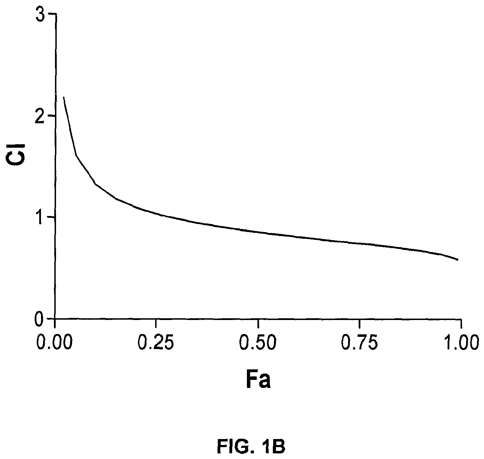

Figure 1B is the Combination Index (CI) effect blot (Fa = fraction effect) for the combination of sodium meta arsenite and cisplatin in A549 cells.

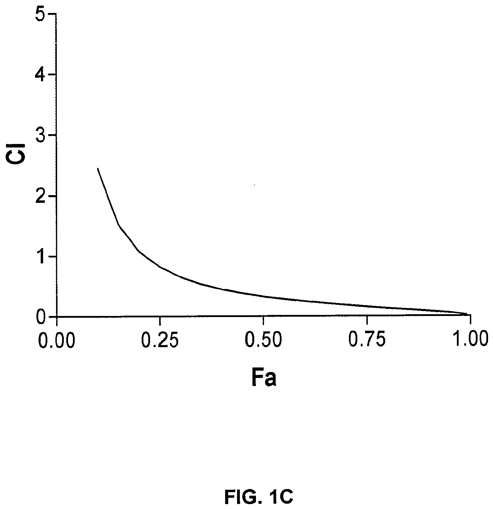

Figure 1C is the Combination Index (CI) effect blot (Fa = fraction effect) for the combination of sodium meta arsenite and cisplatin in H460 cells. CI< 1 demonstrates synergy. C = 1 is additive; C>1 is antagonistic.

Use of a kit, composition or method (*e.g*., combination therapy) described herein results in a synergetic anti-cancer effect to achieve maximum therapeutic benefit, and can improve tolerance to the therapy with a reduced risk of side effects that often result from use of higher doses or longer term monotherapies (*i.e.,* therapies with each compound alone). Therefore, the compositions, kits and methods described herein may enable the use of lower doses of each compound (*e.g.*, lower doses of the arsenic compound or cisplatin) with reduced adverse effects of each compound (*e.g.*, reduced side effects of arsenic compounds or cancer medicaments such as cisplatin). Suboptimal dosages can provide increased safety margins, and can also reduce the costs of drug(s) necessary to achieve prophylaxis and therapy. A synergistic treatment utilizing a combination of the arsenic compound(s) and cisplatin, adriamycin, docetaxel and/or paclitaxel or other taxane can also provide increased convenience and may result in enhanced compliance. Advantages of a combination therapy can additionally include higher stability towards degradation and metabolism, longer duration of action, and/or longer duration of action or effectiveness at particularly low doses.

Methods for treating cancer described herein comprise administering to a mammal in need of such treatment an effective amount of the arsenic compound(s) in combination with cisplatin or another cytotoxic anti-cancer agent that inhibits or interferes with telomeres, such as a taxane. The arsenic compound or combination of arsenic compounds may be administered either prior to, after or concurrently with administration of cisplatin or other such cytotoxic anti-cancer agent. For example, a daily dosage of the arsenic compound can be administered for one to ten days, followed by a dosage regimen of the cytotoxic agent(s) of one to ten days, or longer as necessary. Alternatively, the cytotoxic agent(s) may be delivered prior to the arsenic compound(s): Also, both the arsenic compound(s) and cytotoxic agent(s) may be administered to the patient concurrently, although not necessarily at the same time. The dosing regimen can readily be ascertained by the treating physician based on such factors as dosages of each of the drugs being administered, type and stage of the cancer, patient health, and the like.

When the arsenic compound is sodium meta arsenite, it may be administered in any form, *e.g.,* orally, *via* infusion, rectally, intraperitoneally, etc. When arsenic trioxide is included in the treatment, it may be administered *via* infusion, but any acceptable administration route may be used. Similarly, cisplatin or other cytotoxic anti-cancer agent, e.g., taxane, is generally administered *via* infusion, but may be administered *via* any acceptable route known in the art.

Described herein are improved methods and products for the treatment of subjects having cancer or at risk of developing cancer or metastasis of a primary tumor. The present invention is based, in part, on the finding that when sodium meta arsenite (NaAsO₂ and/or arsenic trioxide is used in conjunction with cisplatin or other teleomere inhibiting cytotoxic anti-cancer agent, such as adriamycin or taxane, such as docetaxel and/or paclitaxel, some unexpected and improved results are observed that indicate synergy between the arsenic and non-arsenic anticancer agents. For instance, the efficacy of the combination therapy is profoundly improved in cancer patients whose tumors have longer telomeres over the use of either of the anticancer agents alone. Also, the toxicity-of the combination therapy may be greatly reduced in cancer patients in comparison to the use of either of the anticancer agents alone.

Also described herein is the use of a telomerase inhibitor other than cisplatin in combination with either sodium meta arsenite or arsenic trioxide. For example, it is contemplated that the combination of one of these arsenic compounds with adriamycin, and/or a toxane such as docetaxel or paclitaxel, which are known to synergize with other telomerase inhibitors or a telomerase binding peptides or small molecules that block and inhibit telomere repair may be used in the kits, compositions and methods described herein. The skilled practitioner can readily determine the dosage amount, formulation and regimen for administration of these telomerase inhibitors based on their known usage in combination with other anti-cancer drugs or usage as single agent anti-cancer therapeutics.

According to the present disclosure, the combination of the arsenic compound(s) and cisplatin and/or other cytotoxic anti-cancer agent, e.g., adriamycin, taxane, *e.g*., docetaxel and/or paclitaxel, enables the effective treatment of cancers that may not be successfully treatable with either drug alone. For example, cisplatin is known to be an effective treatment for some lung cancers, especially those with long telomeres, while sodium meta arsenite is more effective in the treatment of cancers with short telomeres, *e.g*., prostate cancer. However, the combination of cisplatin and sodium meta arsenite is surprisingly significantly more effective in the treatment of tumor cells with long telomeres, *i.e.,* certain lung cancers, than cisplatin alone. Accordingly, described herein are compositions and methods for the synergistic treatment of a variety of solid tumors and/or malignant diseases or conditions, including, but not limited to, the following:
carcinoma including that of the bladder (including accelerated and metastatic bladder cancer), breast, cervical, colon (including colorectal cancer), kidney, liver, lung (including small and 1 lung cancer and lung adenocarcinoma), ovary, prostate, testes, genitourinary tract, lymphatic system, rectum, larynx, pancreas (including exocrine pancreatic carcinoma), esophagus, stomach, gall bladder, cervix, thyroid, and skin (including squamous cell carcinoma);
hematopoietic tumors of lymphoid lineage including leukemia, acute lymphocytic leukemia, acute lymphoblastic leukemia, B-cell lymphoma, T-cell lymphoma, Hodgkins lymphoma, non-Hodgkins lymphoma, hairy cell lymphoma, histiocytic lymphoma, and Burketts lymphoma; hematopoietic tumors of myeloid lineage including acute and chronic myelogenous leukemias, myelodysplastic syndrome, myeloid leukemia, and promyelocytic leukemia; tumors of the central and peripheral nervous system including astrocytoma, neuroblastoma, glioma, and schwannomas; tumors of mesenchymal origin including fibrosarcoma, liposarcoma, rhabdomyosarcoma, and osteosarcoma; and other tumors including melanoma, xenoderma pigmentosum, keratoactanthoma, seminoma, thyroid follicular cancer, and teratocarcinoma.

In certain embodiments described herein, cisplatin, adriamycin, and/or taxane, such as larotaxel, orataxel, tesetaxel, docetaxel and/or paclitaxel is administered in the treatment of a patient who is being subjected to a treatment regime of sodium meta arsenite and/or arsenic trioxide. In certain embodiments, the patient undergoing such treatment has lung cancer or breast cancer or ovarian cancer. In other embodiments, cisplatin is administered in the treatment of a patient who is being subjected to a treatment regime of sodium meta arsenite.

Further described herein are compositions, kits and methods for the treatment of a variety of solid tumors and leukemias including, but not limited to, those tumors listed in Table 1.

**Table 1.**

| | | |
|---|---|---|
| Fibrosarcoma | colon cancer | adenocarcinoma |
| myxosarcoma | colorectal cancer | sweat gland carcinoma |
| Liposarcoma | kidney cancer | sebaceous gland carcinoma |
| chondrosarcoma | pancreatic cancer | papillary carcinoma |
| osteogenic sarcoma | bone cancer | papillary adenocarcinomas |
| Chordoma | breast cancer | cystadenocarcinoma |
| angiosarcoma | ovarian cancer | papillary adenocarcinomas |
| endotheliosarcoma | prostate cancer | cystadenocarcinoma |
| lymphangiosarcoma | esophogealcancer | medullary carcinoma |
| lymphangioendotheliosarcoma | stomach cancer | bronchogenic carcinoma |
| Synovioma | oral cancer | renal cell carcinoma |
| Mesotheliorria | nasal cancer | hepatoma |
| Ewing's tumor | throat cancer | bile duct carcinoma |
| leiomyosarcoma | squamous cell carcinoma | choriocarcinoma |
| rhabdomyosarcoma | basal cell carcinoma | seminoma |
| embryonal carcinoma | lung cancer | craniopharyngioma |
| Wilms' tumor | epithelial carcinoma | ependymoma |
| Cervical cancer | Glioma | pinealoma |
| uterine cancer | Glioblastoma multiforme | hemangioblastoma |
| testicular cancer | Astrocytoma | acoustic neuroma |
| small cell lung carcinoma | medulloblastoma | oligodendroglioma |
| bladder carcinoma | skin cancer | meningioma |
| retinoblastoma | Melanoma | bronchogenic carcinoma |
| Medullary carcinoma | neuroblastoma | |

The present invention provides compositions and kits for use in the treatment of lung cancer. Most lung cancers exhibit striking chromosomal abnormalities and overexpression of certain oncogenes. Lung cancer can be histologically subclassified into squamous cell carcinoma, small cell carcinoma, adenocarcinoma, large cell carcinoma (non-small cell carcinoma), carcinoid tumor, mesothelioma, etc.

In certain embodiments described herein, the kits and compositions may further comprise another therapeutic agent useful for the treatment of a particular cancer or tumor. For example the kits, compositions and methods may include one or more optional treatment agents, such as immunotherapeutic agents, cancer vaccines, biological response modifiers (*e.g*., cytokines and hemopoietic growth factors), or hormone therapies (*e.g*., adrenocorticosteroids, androgens, anti-androgens, estrogens, anti-estrogens, progestins, aromatase inhibitor, gonadotropin-releasing hormone agonists, somatostatin analog; and the like).

In still another embodiment, the optional treatment agent is a hormone, such as, when appropriate, an estrogen therapy e.g., diethylstilbestrol and ethinyl estradiol, anti-estrogen therapy *e.g.,* tamoxifen, progestin therapy *e.g.,* medroxyprogesterone and megestrol acetate, androgen blockade *e.g*., anti-androgens such as flutamide, adrenocorticosteroids including adrenal steroids, synthetic glucocorticoid therapy *e*.*g.*, prednisone, methylprednisone, and dexamethasone, androgens e.g., fluoxymesterone, synthetic testosterone analogs, aromatase inhibitor *e.g.,* aminoglutethimide, gonadotropin-releasing hormone agonists *e.g.,* leuprolide, somatostatin analogs *e.g*., octreotide. In certain embodiments, the method further comprises administering interferon-α to the subject.

As used herein, the term "pharmaceutical composition" refers to either a mixture or combination of an arsenic compound selected from arsenic trioxide, sodium meta arsenite and combinations thereof, and cisplatin, adriamycin, docetaxel, paclitaxel or combinations thereof or each of the selected compounds alone. A pharmaceutical composition can include other chemical components, such as physiologically/pharmaceutically acceptable carriers and excipients.

As used herein, "treating" or "treatment" refers to the administration of two or more agents (*i.e.,* an arsenic compound or arsenic compounds and cisplatin, adriamycin, docetaxel, paclitaxel, or combination thereof or other cytotoxic anti-cancer agent) to a subject having or at risk of developing cancer or metastasis. "Inhibition of the growth of tumor cells" refers to the inhibition of the growth of tumor cells present in the patient. Such treatment may also lead to the regression of tumor growth, *i.e.,* the decrease in size of a measurable tumor. In some embodiments of the invention, such treatment leads to the complete regression of the tumor.

The term "prevention" includes either preventing the onset of clinically evident neoplasia altogether or preventing the onset of a preclinically evident stage of neoplasia in individuals at risk, *e.g*., metastasis. Also intended to be encompassed by this definition is the prevention of initiation for malignant cells or to arrest or reverse the progression of premalignant cells to malignant cells. This includes prophylactic treatment of those at risk of developing the neoplasia.

As used herein, the term "synergistic result" or "synergy" refers to a therapeutic effect against a particular cancer such that when administered in combination, the arsenic compound and cisplatin or other cytotoxic agent described herein produce results that are significantly better than the optimal efficacy obtained with either single agent alone in the treatment of the cancer.

As used herein, "administration" or "administer" or "administering" refers to dispensing, applying, or tendering two or more agents (for example sodium meta arsenite and/or arsenic trioxide and cisplatin, adriamycin and/or taxane, *e.g*., paclitaxel or docetaxel) to a subject. Administration can be performed using any of a number of methods known in the art. For example, "administering" as used herein is meant *via* infusion (intravenous administration (i.v.)), parenteral and/or oral administration. By "parenteral" is meant intravenous, subcutaneous and intramuscular administration. In the use of the subject invention, sodium meta arsenite, for example, can be administered simultaneously with, for example, cisplatin, or the compounds can be administered sequentially, in either order. It will be appreciated that the actual preferred method and order of administration will vary according to, *inter alia,* the particular formulation of arsenic compound being utilized; the particular formulation of ther cytotoxic anti-cancer agent (*e.g*., cisplatin, adriamycin, and/or taxane) being utilized; the particular tumor cells being treated and the particular host being treated. The method and order of administration of an arsenic compound(s) and other anti-cancer medicament(s) for a given set of conditions can be ascertained by those skilled in the art using conventional techniques and in view of the information set out herein.

The arsenic compound(s) described herein and cisplatin, adriamycin, docetaxel, paclitaxel and/or other cytotoxic anti-cancer agent can be administered as part of a combination therapy or co-therapy. Therefore, "combination therapy" (or "co-therapy") embraces the administration of the arsenic compound(s) and cisplatin or other cytotoxic anti-cancer agent described herein as part of a specific treatment regimen intended to provide a beneficial effect from the synergistic co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" generally is not intended to encompass the administration of two or more of these therapeutic agents as part of separate monotherapy regimens that incidentally and arbitrarily result in the combinations described herein. "Combination therapy" is intended to embrace administration of these therapeutic agents in a sequential manner or simultaneously, that is, wherein each therapeutic agent is administered at a different time, as well as administration of these therapeutic agents, or at least two of the therapeutic agents, in a substantially simultaneous manner.

Sequential or substantially simultaneous administration of each therapeutic agent can be effected by any appropriate route including, but not limited to, oral routes, intravenous routes, intramuscular routes, direct absorption through mucous membrane tissues, and combinations thereof. The therapeutic agents can be administered by the same route or by different routes. For example, a first therapeutic agent of the combination selected can be administered by intravenous injection, *e.g.,* cisplatin or arsenic trioxide, while the other therapeutic agent, *e.g.,* sodium meta arsenite can be administered orally. Alternatively, for example, both or all therapeutic agents can be administered by intravenous injection or infusion. The sequence in which the therapeutic agents are administered is not critical.

"Combination therapy" also can embrace the administration of the therapeutic agents as described above in further combination with other biologically active ingredients (such as, but not limited to, a different antineoplastic agent) and non-drug therapies (such as, but not limited to, surgery or radiation treatment). Where the combination therapy further comprises radiation treatment, the r adiation treatment can be conducted at any suitable time so long as a beneficial effect from the co-action of the combination of the therapeutic agents and radiation treatment is achieved. For example, in appropriate cases, the beneficial effect is still achieved when the radiation treatment is temporally removed from the administration of the therapeutic agents, perhaps by days or even weeks.

The term "therapeutically effective" is intended to qualify the amount of each agent that will achieve the goal of improvement in tumor size or malignant or neoplastic disease severity and the frequency of neoplastic disease over treatment of each agent by itself, while avoiding adverse side effects typically associated with alternative therapies. A "therapeutic effect" or "therapeutic effective amount" is also intended to qualify the amount of an anticancer agent required, in combination or composition with one or more other anticancer agent, to relieve to some extent one or more of the symptoms of a neoplasia disorder, including, but is not limited to: 1) reduction in the number of cancer cells; 2) reduction in tumor size; 3) inhibition (i.e., slowing to some extent, or stopping) of cancer cell infiltration into peripheral organs; 3) inhibition (i.e., slowing to some extent, or stopping) of tumor metastasis; 4) inhibition, to some extent, of tumor growth; 5) relieving or reducing to some extent one or more of the symptoms associated with the disorder; and/or 6) relieving or reducing the side effects associated with the administration of anticancer agents.

Described herein is a pharmaceutical composition, kit and method for combination therapy treatment or prevention and inhibition of the growth of solid tumors, leukemias or metastasis, which involves the administration of sodium meta arsenite and/or arsenic trioxide with cisplatin, adriamycin, docetaxel, paclitaxel and/or other cytotoxic anti-cancer agent in effective amounts to a subject in need of treatment. In certain embodiments, described herein is a pharmaceutical composition, kit and method including combination therapy for treatment of lung cancer or prevention of the metastasis of lung cancer to other sites or organs in the patient.

Also described herein is a kit comprising sodium meta arsenite and/or arsenic trioxide and cisplatin, adriamycin, docetaxel, paclitaxel, and/or other cytotoxic anti-cancer agent together in a composition or separate compositions for a combination therapy. The kit can be a package which houses a container or containers that contain the arsenic compound(s) and cisplatin, adriamycin, docetaxel, paclitaxel and/or other cytotoxic anti-cancer agent, and also houses instructions for administering the composition(s) to a subject. In particular, a kit can comprise instructions for simultaneous, separate or sequential use. A kit can contain a single dosage form or it can contain separate dosage forms, *i.e*. one for each therapeutic agent to be administered. In one embodiment, the kit comprises a fixed ratio dosage of the arsenic compound(s) and cisplatin or other cytotoxic anti-cancer agent. The kit can also contain multiple doses of each of the anti-cancer agents, whether combined or separately formulated.

The kit can additionally include other materials desirable from a commercial and user standpoint, including, without limitation, buffers, diluents, filters, needles, syringes, and package inserts with instructions for performing any methods disclosed herein (e.g., methods for treating a disease disclosed herein). A medicament or formulation in a kit described herein can comprise any of the combinations, or compositions disclosed herein.

The kits described herein can be useful for any of the methods disclosed herein including, without limitation, treating a subject having or at risk of developing cancer, solid tumor, or lung cancer.

### Formulation

The active ingredients described herein are formulated into pharmaceutical preparations (e.g., together in a composition or separately to be used in a combination therapy) for administration to mammals for treatment of cancer.

For oral administration, the pharmaceutical preparation can be in liquid form, for example, solutions, syrups or suspensions, or can be presented as a drug product for reconstitution with water or other suitable vehicle before use. Such liquid preparations can be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, or fractionated vegetable oils); and preservatives (e.g., methyl or propyl-p-hydroxybenzoates or sorbic acid). The pharmaceutical compositions can take the form of, for example, tablets or capsules prepared by conventional means with pharmaceutically acceptable excipients such as binding agents (e.g., pregelatinized maize starch, polyvinyl pyrrolidone or hydroxypropyl methylcellulose); fillers (e g., lactose, microcrystalline cellulose or calcium hydrogen phosphate); lubricants (e. g., magnesium stearate, talc or silica); disintegrants (e.g., potato starch or sodium starch glycolate); or wetting agents (e.g., sodium lauryl sulphate). The tablets can be coated by methods well-known in the art.

Preparations for oral administration can be suitably formulated to give controlled release of the active compound. For oral administration, the compositions can take the form of tablets or lozenges formulated in conventional manner.

For administration by inhalation, the compounds for use according to the present disclosure are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The therapeutic agents can be formulated for parenteral administration by injection, e.g., by bolus injection or continuous infusion. Such formulations are sterile. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

The compounds can also be formulated in rectal compositions such as suppositories or retention enemas, e.g., containing conventional suppository bases such as cocoa butter or other glycerides.

In addition to the formulations described previously, the compounds can also be formulated as a depot preparation. Such long acting formulations can be administered by implantation (for example, subcutaneously or intramuscularly) or by intramuscular injection. Thus, for example, the compounds can be formulated with suitable polymeric or hydrophobic materials (for example, as emulsion in acceptable oils) or ion exchange resins, or as sparingly soluble derivatives, for example, as a sparingly soluble salt. Liposomes and emulsions are well known examples of delivery vehicles or carriers for hydrophilic drugs.

The pharmaceutical preparations can, if desired, be presented in a pack or dispenser device which can contain one or more unit dosage forms containing the active ingredient. The pack can for example comprise metal or plastic foil, such as a blister pack. The pack or dispenser device can be accompanied by instructions for administration.

### Method of Administration

It will be recognized by one of skill in the art that the content of the active ingredients in the pharmaceutical preparations described herein can vary quite widely depending upon numerous factors such as, the desired dosage and the pharmaceutically acceptable carrier being employed. For administration, the dosage amount of the arsenic compound will usually be in the range of from about 0.1 mg/kg to about 100 mg/kg, in certain embodiments from about 1.0 to about 50 mg/kg, in other embodiments from about 2.5 to about 25 mg/kg, and in other embodiments from about 3 to about 15 mg/kg.

In some embodiments, the cisplatin-containing pharmaceutical compositions described herein will contain cisplatin in an amount from about 0.1 mg/ml to about 500 mg/ml, or about 1 mg/ml to about 50 mg/ml, and in other embodiments, from about 1 to 5 mg/ml. Mannitol and/or sodium chloride can be included in amounts conventional for cisplatin preparations. Physiological pH of injectables or infusion drug combinations will be established by inclusion of buffering agents as is known in the art.

When other cytotoxic anti-cancer agents are used in place of cisplatin, the amount is determined on the basis of the properties of the agent used. For example, adriamycin may be administered at a dose of 60 mg/m², administered as a continuous infusion through a central venous catheter or for example, as a 15- or 25-day courses of ADM at a mean dose of about 3.8 mg/m² (2.2-4.5 mg/m²) infused by programmable portable pump. Similarly, docetaxel or paclitaxel (or other taxane) can be given as a high-dose chemotherapeutic agent, for example, 250 mg/m² once every three weeks, once every two weeks, or in low doses, less than 100 mg/m² on a weekly basis, for example. In some cases, a taxane, *e.g*., docetaxel or paclitaxel may be given slowly during a 24-hour infusion. The skilled practitioner can determine the appropriate dose of anti-cancer agent and time course of delivery depending on several factors including the relative health of the patient and the type and stage of the cancer, as well as the other drugs administered in the combination therapy.

The appropriate pharmaceutically acceptable carriers and diluents to be utilized in the pharmaceutical preparations described herein are well known to those skilled in the art of formulating compounds into pharmaceutical compositions. The pharmaceutical preparations described herein that are in a form suitable for parenteral administration can be formulated for intravenous infusion or injection in numerous ways well known to those skilled in the art with pharmaceutically acceptable carriers. In certain embodiments, such pharmaceutical preparations are in the form of a freeze-dried mixture of the active ingredients in a unit dosage form, prepared by conventional techniques, which can be reconstituted with water or other suitable infusion liquid at the time of administration.

For the treatment of breast cancer and many other forms of solid tumors, as well as in treatment of leukemias and lung cancer, the arsenic compounds described herein are more likely to be administered systemically, in a pharmaceutical composition containing such excipients or inert components, which are well known in the art pertaining to chemotherapy of tumors. More specifically, if an arsenic compound is to be administered systemically, it can be confected as a powder, pill, tablet or the like or as a syrup or elixir suitable for oral administration. For intravenous or intraperitoneal administration, the arsenic compound will be prepared as a solution or suspension capable of being administered by injection. In certain cases, it can be useful to formulate these compounds by injection. In certain other cases, it can be useful to formulate these compounds in suppository form or as extended release formulation for deposit under the skin or intramuscular injection.

The arsenic compound described herein will be administered as a chemotherapeutic agent together with cisplatin in a useful therapeutic dose which will vary from condition to condition and which, in certain instances can vary with the severity of the condition being treated and the patients susceptibility to treatment. Accordingly, no single dose will be uniformly useful, but will require modification depending on the particularities of the tumor or malignancy being treated. Such doses can be arrived at through routine experimentation. For the treatment of solid tumors and leukemias, particularly breast cancer and acute myeloid leukemia, it is anticipated that the arsenic compounds described herein will be administered for approximately 1 to 8 weeks to a patient in need thereof, in a dose that is effective to halt, slow the growth or dissipate the tumor or halt leukemia cell proliferation. In certain embodiments, the arsenic compound is sodium meta arsenite, which is to be administered orally, in a daily dose which in some embodiments described herein will be in the range of an approximately 0.0001 mg/kg per day to 100 mg/kg per day; or in the range of 0.05 mg/kg to 50 mg/kg per day; or in other embodiments, in the range of 1 mg to 25 mg per day; and in other embodiments in the range of 2 mg/kg to 20 mg/kg per day or 2.5 to 10 mg/kg per day. However, the dose can be adjusted upward or downward to suit the needs of any particular patient.

The cisplatin or other cytotoxic anti-cancer agent component of the combination therapy can be administered in accordance with the present disclosure in the same manner as known in clinical practice. For example, slow intravenous infusion is the method of choice for cisplatin. For promoting diuresis when using cisplatin, the incorporation of mannitol in a dextrose/saline solution is the preferred carrier. The protocol can also include prehydration of the patient by administration of a dextrose/saline solution before administration of the cisplatin, adriamycin, docetaxel and/or paclitaxel. In some embodiments described herein, the dosage of cisplatin, when administered with an arsenic compound, is a single dose of from about 3 to about 100 mg/m² of cisplatin, and in certain embodiments is delivered at the end of a one to five consecutive day course of treatment with the arsenic compound. In other embodiments, the dose of cisplatin is about 20 mg/m² or more once every three to four weeks. Alternatively, therapeutically appropriate amounts of other cytoxic agents, e.g., adriamycin, docetaxel and/or paclitaxel can be administered to the patient. Infusions of cisplatin, adriamycin, docetaxel and/or paclitaxel or other cytotoxic agent can be given one to two times weekly, and the weekly treatments repeated several times unless renal toxicity, neurotoxicity or other side effects provide a contraindication.

For parenteral administration of arsenic trioxide, the course of therapy generally employed is from 0.0001 to 100 mg/kg per day for about five consecutive days, or 0.001 to 50 mg/ml, and in other embodiments, 0.01 to 20 mg/kg for about five consecutive days. In some embodiments described herein, the dosage range of arsenic trioxide to be used in combination with cisplatin is about 0.1 to 5.0 mg/kg per day. The skilled practitioner can determine the appropriate amount and course for the administration of arsenic trioxide and adjust the treatment protocol accordingly.

### EXAMPLE 1:

***In vitro Studies with cisplatin and sodium meta arsenite.*** Both cisplatin and sodium meta arsenite can cause telomere, damage. Therefore, the combination of these two anti-cancer agents was tested to determine whether the two drugs show evidence of *in vitro* synergy in two non-small cell lung cancer cell lines. H460 (4kb, IC50, IC50=10µM) and A549 (6kb, IC50=13µM) were chosen, because they have relatively short telomeres and are part of a National Cancer Institute (NCI) 60 cell line panel. IC50 concentrations for cisplatin and KML001 were determined by MTT assay and the widely accepted median effect methodology by Chou and Talalay (Advances in Enzyme Regulation (1984), 22:27-55, incorporated in its entirety herein), based on fixed IC50 ratios used for determination of synergy, additivity or antagonism (Fig. 1A-1C).

***Cell culture and MTT assay.*** Cells were grown under standard conditions (5% CO₂,/37°C/humidified atmosphere) in their respective recommended media such as RMPI 1640, Iscove's or DMEM (Invitrogen) and passaged routinely. For MTT proliferation assays, exponentially growing cells were harvested and plated in 96-well plates (2,000/well). To assess the growth inhibitory potential of drugs, test drugs were added at concentrations ranging from 1 nM to 100 µM. To determine cell growth at the time of drug addition (d0), which enables calculation of the actual cell kill, one of the 96-well plates was immediately developed after drug addition. The others were incubated for 5 days before 3-(4,5-dimethylthiazol-2-yl)-2,5-diphenyltetrazoliumbromide (MTT) was added and the conversion to purple formazan by viable cells was measured using a SynergyHT plate reader (550 nm) and K4C software (BioTEK). Formazan was dissolved with DMSO. Growth curves were generated in MSExcel and growth inhibitory concentration 50 and 100% as well as net cell kill were determined. The results are presented in Figures 1A-C and Tables 2-4.

A549 and H460 have an IC50 for cisplatin of 1.5 and I µM respectively, but are relatively insensitive to sodium meta arsenite although H460 cells, which have shorter telomeres (4 kb) have a lower IC50 than A549 cells with relatively longer telomeres (6 kb, Fig. 1A). When the two drugs were combined, however, a very marked synergism was obtained for most levels of effect (Fig. 1B-C, Tables 2-4). Combination indices (CI) of well below 1 were found for the effective dose (ED) levels 50, 75 and 90 % (Table 2), reducing, *e.g*., the IC50 of cisplatin from 1.5 µM to 0.45 µM in A549 cells (Fig. 1A). Thus, KML001 is able to sensitize lung cancer cell lines to cisplatin.

**Table 2 Combination Index Values**

| **Cell Line** | **CI ED 50** | **CI ED75** | **CI ED90** |
|---|---|---|---|
| A549 | 0.86 | 0.75 | 0.68 |
| H460 | 0.33 | 0.16 | 0.09 |

**Table 3 Single Agent Index Values: H460 Cells**

| **µM** | **Sodium Meta Arsenite** | **Cisplatin** | **Sodium Meta Arsenite-Cisplatin** |
|---|---|---|---|
| IC50 | 10 | 1 | 0.7 |
| IC75 | 20 | 1.7 | 1 |
| IC90 | 30 | 2.5 | 1.5 |

**Table 4 Single Agent Index Values: A549 Cells**

| **µM** | **Sodium Meta Arsenite** | **Cisplatin** | **Sodium Meta Arsenite-Cisplatin** |
|---|---|---|---|
| IC50 | 13 | 1.5 | 0.457 |
| IC75 | 19 | 2 | 0.7 |
| IC90 | 17 | 4 | 1.1 |

## Claims

1. A kit for use in the treatment of lung cancer comprising a first composition comprising a therapeutically effective amount of sodium meta arsenite and a second composition comprising a therapeutically effective amount of a cytotoxic anti-cancer agent selected from the group consisting of cisplatin, paclitaxel and docetaxel.

2. The kit for use according to claim 1 wherein the first composition comprises sodium meta arsenite and the second composition comprises cisplatin.

3. The kit for use according to claim 2 wherein the first composition is formulated for oral administration.

4. The kit for use according to claim 1 wherein the first composition comprises sodium meta arsenite and the second composition comprises paclitaxel.

5. An anti-cancer agent selected from the group consisting of cisplatin, paclitaxel and docetaxel for use in the treatment of lung cancer in a human patient, wherein the patient is being, has been or will be subjected to a treatment regime comprising the administration to the patient of sodium meta arsenite.

6. The anti-cancer agent for use according to claim 5 wherein the anti=cancer agent is cisplatin.

7. The anti-cancer agent for use according to claim 5 wherein the anti-cancer agent is paclitaxel.

## Patentansprüche

1. Kit zur Verwendung in der Behandlung von Lungenkrebs, umfassend eine erste Zusammensetzung, umfassend eine therapeutisch wirksame Menge von Natriummetaarsenit, und eine zweite Zusammensetzung, umfassend eine therapeutisch wirksame Menge an cytotoxischem Antikrebsmittel, ausgewählt aus der Gruppe, bestehend aus Cisplatin, Paclitaxel und Docetaxel.

2. Kit zur Verwendung gemäß Anspruch 1, wobei die erste Zusammensetzung Natriummetaarsenit umfaßt und die zweite Zusammensetzung Cisplatin umfaßt.

3. Kit zur Verwendung gemäß Anspruch 2, wobei die erste Zusammensetzung zur oralen Verabreichung formuliert ist.

4. Kit zur Verwendung gemäß Anspruch 1, wobei die erste Zusammensetzung Natriummetaarsenit umfaßt und die zweite Zusammensetzung Paclitaxel umfaßt.

5. Antikrebsmittel, ausgewählt aus der Gruppe, bestehend aus Cisplatin, Paclitaxel und Docetaxel, zur Verwendung in der Behandlung von Lungenkrebs bei einem humanen Patienten, wobei der Patient einer Behandlung, umfassend die Verabreichung von Natriummetaarsenit dem Patienten, unterworfen wird, unterworfen worden ist oder unterworfen werden wird.

6. Antikrebsmittel zur Verwendung gemäß Anspruch 5, wobei das Antikrebsmittel Cisplatin ist.

7. Antikrebsmittel zur Verwendung gemäß Anspruch 5, wobei das Antikrebsmittel Paclitaxel ist.

## Revendications

1. Kit pour une utilisation dans le traitement du cancer du poumon comprenant une première composition contenant une quantité thérapeutiquement efficace de méta-arsénite de sodium et une seconde composition contenant une quantité thérapeutiquement efficace d'un agent anticancéreux cytotoxique choisi dans le groupe constitué de cisplatine, paclitaxel et docétaxel.

2. Kit pour une utilisation selon la revendication 1, dans lequel la première composition contient du méta-arsénite de sodium et la seconde composition contient du cisplatine.

3. Kit pour une utilisation selon la revendication 2, dans lequel la première composition est formulée pour une administration orale.

4. Kit pour une utilisation selon la revendication 1, dans lequel la première composition contient du méta-arsénite de sodium et la seconde composition contient du paclitaxel.

5. Agent anticancéreux choisi dans le groupe constitué de cisplatine, paclitaxel et docétaxel pour une utilisation dans le traitement du cancer du poumon chez un patient humain, où le patient est, a été ou sera soumis à un régime thérapeutique comprenant l'administration au patient de méta-arsénite de sodium.

6. Agent anticancéreux pour une utilisation selon la revendication 5, où l'agent anticancéreux est le cisplatine.

7. Agent anticancéreux pour une utilisation selon la revendication 5, où l'agent anticancéreux est le paclitaxel.
